# EUROPEAN PATENT APPLICATION

(11) **EP 2 737 853 A1**
(43) Date of publication of application: **04.06.2014**
(21) Application number: 13190085.4
(22) Date of filing: 24.10.2013
(51) Int. Cl.: A61B 6/00, A61B 5/00, G06F 19/00, A61B 5/055, A61B 6/03, A61B 8/00

(54) **Method of displaying medical image acquisition information and medical image display apparatus**

(30) Priority: 28.11.2012 US 201261730682 P; 15.04.2013 KR 20130041256
(71) Applicant: Samsung Electronics Co., Ltd, Gyeonggi-do 443-742 (KR)
(72) Inventor: Lee, Ja-youn, Eunpyeong-gu (KR); Kim, Na-ri, Seoul (KR); Lee, Olivia, Seoul (KR); Lee, Jong-kee, Seoul (KR)
(74) Representative: Grünecker, Kinkeldey, Stockmair & Schwanhäusser

(57) **Abstract**

A method of displaying information relating to medical image acquisition includes: identifying execution procedures for acquiring medical images of an object; generating a state display bar including buttons relating to the execution procedures; and displaying the state display bar on a screen. The buttons are arranged on the state display bar and one of the buttons corresponding to one of the execution procedures has a length that is different from lengths of all or some of other buttons corresponding to other execution procedures.

## Description

### CROSS-REFERENCE TO RELATED PATENT APPLICATIONS

This application claims priority from Korean Patent Application No. 10-2013-0041256, filed on April 15, 2013, in the Korean Intellectual Property Office, and US Provisional Application No. 61/730,682, filed on November 28, 2012, the disclosures of which are incorporated herein in their entirety by reference.

### BACKGROUND

### 1. Field

Apparatuses and methods consistent with exemplary embodiments relate to displaying medical image acquisition information, and more particularly, to displaying information relating to a plurality of execution procedures for acquiring medical images.

### 2. Description of the Related Art

A medical image system may include a medical image acquisition apparatus and a medical image display apparatus. The medical image acquisition apparatus sends a predetermined signal to an object, and acquires medical images such as tomograms or blood flow images of the object by using signals reflected by or transmitted through the object.

For example, the medical image acquisition apparatus may acquire ultrasonic images, X-ray images, computed tomography (CT) images, magnetic resonance (MR) images, and/or positron emission tomography (PET) images.

The medical image acquisition apparatus may display acquired medical images on a screen. The medical image display apparatus may be an additional apparatus separate from the medical image acquisition apparatus, an apparatus included in the medical image acquisition apparatus, or an apparatus connected to the medical image acquisition apparatus. The medical image display apparatus may include a console for controlling the medical image acquisition apparatus.

In a medical image acquisition system, a medical image display apparatus may display information relating to acquisition of medical images of an object.

Since resources of the medical image display apparatus are limited, the information amount that the medical image display apparatus may display is also limited. The amount of information that a user may recognize at once is limited.

Therefore, there is a need to provide a user with the necessary information from a large amount of information relating to the medical image acquisition.

### SUMMARY

Exemplary embodiments may address at least the above problems and/or disadvantages and other disadvantages not described above. The exemplary embodiments are not required to overcome the disadvantages described above, and an exemplary embodiment may not overcome any of the problems described above.

One or more of exemplary embodiments provide a method of displaying information about medical image acquisition and a medical image display apparatus, whereby a user may be effectively provided with appropriate information.

One or more of exemplary embodiments also provide a method of displaying information about medical image acquisition for providing information relating to the medical image acquisition based on the order of executing a plurality of processes for acquiring medical images so that a user may rapidly and precisely recognize information, and a medical image display apparatus.

According to an aspect of an exemplary embodiment, there is provided a method of displaying information relating to medical image acquisition by a medical image display apparatus, the method including: identifying a plurality of execution procedures for acquiring medical images about an object; generating a state display bar relating to the plurality of execution procedures; and displaying the state display bar on a screen, wherein a plurality of buttons for displaying medical image acquisition information relating to the plurality of execution procedures are arranged on the state display bar, and a button corresponding to at least one execution procedure has a length that is different from lengths of the other buttons corresponding to the other execution procedures.

The at least one execution procedure may include an execution procedure selected by a user. The at least one execution procedure may include the execution procedure selected by the user and a predetermined number of execution procedures adjacent to the selected execution procedure.

The at least one execution procedure may include an execution procedure that is currently performed by a medical image acquisition apparatus that is connected to the medical image display apparatus. The at least one execution procedure may include the execution procedure that is currently performed and a predetermined number of execution procedures adjacent to the execution procedure that is currently performed.

The state display bar may include a time display bar for displaying a total time of the execution procedure that is currently performed. The state display bar may include a region for displaying a remaining time until the execution procedure that is currently performed is finished.

The state display bar may include an entire time display bar for displaying at least one of a total time for performing the plurality of execution procedures, an elapsed time from the beginning of the plurality of execution procedures to the execution procedure that is currently performed, and a remaining time until the plurality of execution procedures are finished.

The state display bar may include icons for guiding operations of the user for acquiring the medical image. The icons may be displayed between buttons corresponding to the execution procedures that are performed before and after the operations of the user.

The button corresponding to the at least one execution procedure may have a different color and shape from those of the buttons corresponding to other execution procedures.

The plurality of buttons may respectively correspond to the plurality of execution procedures, and the plurality of buttons arranged on the state display bar may be displayed on one screen.

The method may further include, when one of the plurality of buttons is selected, displaying medical image acquisition information corresponding to the selected button on the screen. The medical image acquisition information displayed on the screen may include at least one of an image acquired in an execution procedure corresponding to the selected button, state information relating to the execution procedure corresponding to the selected button, and a user interface relating to the execution procedure corresponding to the selected button.

According to another aspect of an exemplary embodiment, there is provided a medical image display apparatus including: an execution procedure identifier for identifying a plurality of execution procedures for acquiring a medical image of an object; a state display bar generator for generating a state display bar relating to the plurality of execution procedures; and a display for displaying the generated state display bar on a screen, wherein a plurality of buttons for displaying medical image acquisition information relating to the plurality of execution procedures are arranged on the state display bar, and a button corresponding to at least one execution procedure has a length that is different from lengths of the other buttons corresponding to the other execution procedures.

The at least one execution procedure may include an execution procedure selected by a user. The at least one execution procedure may include the execution procedure selected by the user and a predetermined number of execution procedures adjacent to the selected execution procedure.

The state display bar may include an entire time display bar for displaying at least one of a total time for performing the plurality of execution procedures, an elapsed time from the beginning of the plurality of execution procedures to the execution procedure that is currently performed, and a remaining time until the plurality of execution procedures are finished.

The state display bar may include icons for guiding operations of the user for acquiring the medical image. The icons may be displayed between buttons corresponding to the execution procedures that are performed before and after the operations of the user.

The button corresponding to the at least one execution procedure may have a different color and shape from those of the buttons corresponding to other execution procedures.

The plurality of buttons may respectively correspond to the plurality of execution procedures, and the plurality of buttons arranged on the state display bar may be displayed on one screen.

When one of the plurality of buttons is selected, medical image acquisition information corresponding to the selected button may be displayed on the screen. The medical image acquisition information displayed on the screen may include at least one of an image acquired in an execution procedure corresponding to the selected button, state information relating to the execution procedure corresponding to the selected button, and a user interface relating to the execution procedure corresponding to the selected button.

According to another aspect of an exemplary embodiment, there is provided a computer-readable recording medium having embodied thereon a computer program for executing the above method on a computer.

### BRIEF DESCRIPTION OF THE DRAWINGS

The above and/or other aspects will become more apparent by describing in certain exemplary embodiments with reference to the accompanying drawings, in which:

FIG. 1 is a diagram showing a medical image system according to an exemplary embodiment;

FIG. 2 is a flowchart illustrating a method of displaying medical image acquisition information, according to an exemplary embodiment;

FIG. 3 is a flowchart illustrating a method of displaying medical image acquisition information based on a user's selection, according to an exemplary embodiment;

FIG. 4 is a flowchart illustrating a method of displaying medical image acquisition information based on a process that is currently executed;

FIG. 5 is a block diagram of a medical image display apparatus according to an exemplary embodiment;

FIGS. 6A and 6B are diagrams showing a status display bar according to the exemplary embodiment;

FIG. 7 is a diagram showing a status display bar for displaying time information, according to the exemplary embodiment;

FIG. 8 is a diagram showing a status display bar including icons for guiding user operations, according to the exemplary embodiment;

FIG. 9 is a diagram showing a screen on which medical image acquisition information is displayed, according to an exemplary embodiment;

FIG. 10 is a diagram showing a status display bar including a notification message of the execution procedure, according to the exemplary embodiment;

FIG. 11 is a diagram showing an example of a graphical user interface (GUI) for identifying a plurality of execution steps for acquiring medical images, according to an exemplary embodiment; and

FIG. 12 is a diagram showing a user interface for changing a status display bar according to an exemplary embodiment.

### DETAILED DESCRIPTION

Certain exemplary embodiments are described in greater detail below with reference to the accompanying drawings.

In the following description, the same drawing reference numerals are used for the same elements even in different drawings. The matters defined in the description, such as detailed construction and elements, are provided to assist in a comprehensive understanding of exemplary embodiments. Thus, it is apparent that exemplary embodiments can be carried out without those specifically defined matters. Also, well-known functions or constructions are not described in detail since they would obscure exemplary embodiments with unnecessary detail.

Throughout the specification, when a part is "connected" to another part, not only denotes a case where the two parts are "directly connected" to each other is denoted, but also a case where the two parts are "electrically connected" to each other with an intervening device therebetween is denoted. In the specification, when a certain part "includes" a certain component, the part may further include another component unless there is a different disclosure. As used herein, the term "and/or" includes any and all combinations of one or more of the associated listed items. Expressions such as "at least one of," when preceding a list of elements, modify the entire list of elements and do not modify the individual elements of the list.

Also, in the specification, the term "object" may indicate a part of a human body. For example, the object may be an organ, such as the liver, the heart, the womb, the breast, the abdomen, or the like, a fetus, or a cross section of the human body.

A medical image of an object may be an ultrasonic image, an X-ray image, a CT image, an MR image, and a PET image, but an exemplary embodiment is not limited to thereto,

In addition, throughout the specification, the term "user" may be a medical practitioner, a nurse, a medical pathologist, a medical image expert, or the like, but an exemplary embodiment is not limited thereto.

Hereinafter, exemplary embodiments will be described in detail.

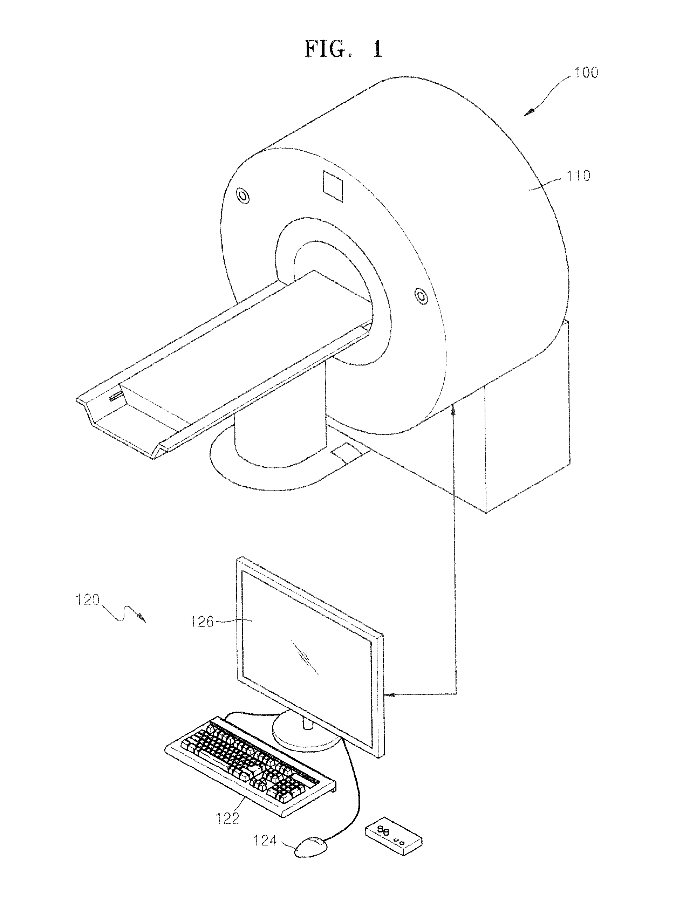

FIG. 1 is a diagram for illustrating a medical image system 100 according to an exemplary embodiment. For example, the medical image system 100 of FIG. 1 may be a CT image system.

As shown in FIG. 1, the medical image system 100 may include a medical image acquisition apparatus 110 and a medical image display apparatus 120.

The medical image acquisition apparatus 110 may include a table supporting an object and a device sending and/or receiving a predetermined signal (for example, X-rays, an MR signal, or ultrasonic waves) to the object. The medical image acquisition apparatus 110 may receive signals generated by the object. The medical image acquisition apparatus 110 may acquire medical images of the object based on the received signals.

The medical image display apparatus 120 may receive the medical images acquired by the medical image acquisition apparatus 110 and display the received medical images. The medical image display apparatus 120 may control the medical image acquisition apparatus 110.

In FIG. 1, the medical image display apparatus 120 is separated from the medical image acquisition apparatus 110; however, an exemplary embodiment is not limited thereto. That is, the medical image display apparatus 120 may be included in the medical image acquisition apparatus 110 or may be connected to the medical image acquisition apparatus 110. The medical image display apparatus 120 may include a console for manipulating the medical image acquisition apparatus 110. The medical image acquisition apparatus 110 and the medical image display apparatus 120 may be connected to each other via a wired or wireless communication network.

The medical image display apparatus 120 may display medical image acquisition information relating to acquisition of the medical images of the object as well as of corresponding medical images. The medical image acquisition information may include information about execution procedures that the medical image system performs for providing the user with the medical images acquired from the object.

For example, information about the execution procedures may include the name of the execution procedures, the total time of each process, state information of the object and the system in the execution procedure, the image of the object acquired in the execution procedure, and the user interface relating to the execution procedure. The medical image system 100 may receive setting values relating to the execution procedures or information about the object, or may output the state of the object or system through the user interface relating to the execution procedure.

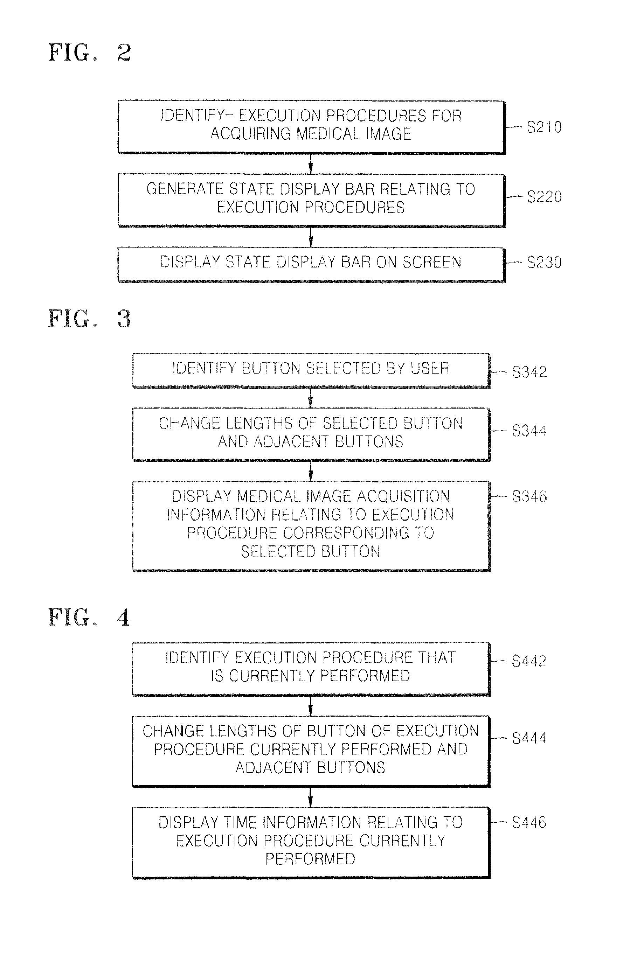

FIG. 2 is a flowchart illustrating a method of displaying medical image acquisition information according to an exemplary embodiment.

In operation S210, the medical image display apparatus identifies a plurality of execution procedures for acquiring medical images of the object.

The plurality of execution procedures to acquire medical images may include execution procedures that the medical image system 100 performs in order to obtain the medical images from the object. For example, the plurality of execution procedures may include execution procedures performed by at least one of the medical image acquisition apparatus 110 and the medical image display apparatus 120. The execution procedures may refer to procedures that a user has to perform.

For example, "a plurality of execution procedures for acquiring a CT image" may only include a scout scan process for setting a scanning range or identifying impurities, and a scan process for obtaining a CT image from the object.

As another example, m order to perform a cardiac scan operation, a contrast medium is injected into a patient and breath of the patient needs to be measured Therefore, m this case, "a plurality of execution procedures for acquiring a CT image" may include the following processes

| Execution procedure | Content |
|---|---|
| Test preparation | Preparing holder according to type of organ (e g head holder, leg holder, etc.) |
| Patient position adjustment | Setting position of organ |
| Patient table adjustment | Adjusting position of table on which the patient lies |
| Contrast medium preparation | Insert contrast medium needle |
| Respiratory setting | Preparing respiratory meter and measuring respiration |
| Light adjustment | On/off manipulation of light (zero point adjustment) |
| Scout Scan | Setting scout technical parameter and perform the scout scan |
| Scan planning | Range setting and series technical parameter setting |
| Pre-contrast | Pre-contrast imaging and re-scan planning |
| Contrast medium injection | Input contrast medium injection command (e g , pushing a button) |
| Post-contrast | Post-contiast imaging |
| Test end | Removing the respiratory meter and contrast medium needle |

When performing a cardiac scan, the pre-contrast process and the post-contrast process may be omitted or may be performed simultaneously according to the medical image acquisition apparatus 110 Thus, the plurality of execution procedures for obtaining medical images are not limited to the above described processes

The plurality of execution procedures for acquiring medical images of the object may be determined based on a user input or based on information stored in a database (DB) 560

For example, when information about the object is input by the user, the medical image display apparatus 120 searches for the DB 560 m order to determine the plurality of execution procedures corresponding to the input information When information about a type of object scanning is input, the medical image display apparatus 120 may search for the DB 560 in order to determine the plurality of execution procedures corresponding to the scanning type. That is, if an imaged part is changed, the plurality of execution procedures for acquiring the medical image may vary. If an imaging protocol is changed even when the same imaging part is imaged, the plurality of execution procedures for acquiring the medical image may also vary.

In operation S220, the medical image display apparatus generates a status display bar relating to the plurality of execution procedures identified in operation S210.

The status display bar generated in operation S220 may include a plurality of buttons relating to the plurality of execution procedures. The plurality of buttons may be formed to display medical image acquisition information relating to the plurality of execution procedures. For example, the plurality of buttons may be arranged in a row on the status display bar according to an order of executing the execution procedures.

The plurality of buttons displayed on the status display bar may respectively correspond to the plurality of execution procedures. For example, a first button corresponding to a first execution procedure, a second button corresponding to a second execution procedure, and a third button corresponding to a third execution procedure may be arranged in a row on the status display bar.

The medical image display apparatus 120 may determine a reference execution procedure from among the plurality of execution procedures for acquiring the medical image.

For example, the reference execution procedure may be set based on a user input for selecting a predetermined execution procedure as the reference execution procedure. The reference execution procedure may be set based on a determination about the execution procedure that is currently performed by the medical image acquisition apparatus 110. The medical image display apparatus 120 may determine as the reference execution procedure one of the execution procedure selected by the user and the execution procedure currently performed.

The medical image display apparatus 120 may display a button corresponding to the reference execution procedure to be different from the other buttons corresponding to the other execution procedures. For example, the button corresponding to the reference execution procedure may have a length that is different from those of the other buttons corresponding to the other execution procedures. Otherwise, the button corresponding to the reference execution procedure may have a color or a shape that is different from those of the other buttons corresponding to the other execution procedures.

The length of the button may be a length of a predetermined side of a polygon, if the button has a polygonal shape. However, an exemplary embodiment is not limited thereto, that is, if the button has a circular shape, the length of the button may be a diameter or a radius of the button.

The medical image display apparatus 120 may display the buttons corresponding to the reference execution procedure and the predetermined number of execution procedures adjacent to the reference execution procedure to be distinguished from the other buttons corresponding to the other execution procedures.

For example, the medical image display apparatus 120 may display the buttons corresponding to the reference execution procedure and the predetermined number of execution procedures adjacent to the reference execution procedure to have lengths that are different from those of the other buttons corresponding to the other execution procedures.

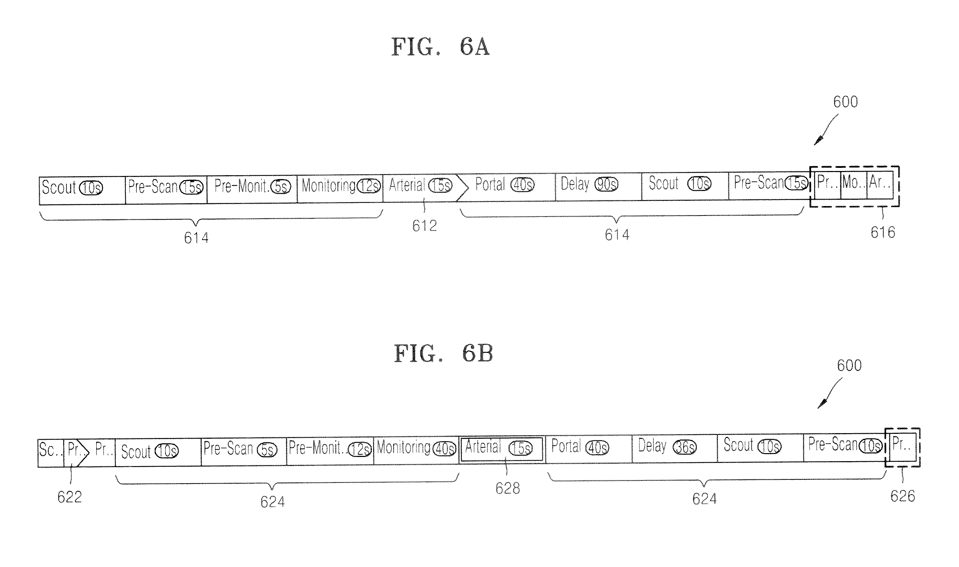

According to the exemplary embodiment, the user may recognize the reference execution procedure from among the plurality of execution procedures based on the length, the shape, or the color of the buttons corresponding to the execution procedures. The state display bar including the plurality of buttons will be described below with reference to FIG. 6.

The state display bar generated by the medical image display apparatus 120 according to the exemplary embodiment may display time information relating to a predetermined execution procedure. The execution procedure of which time information is displayed may be the execution procedure that is currently performed by the medical image system 100 or the execution procedure selected by the user.

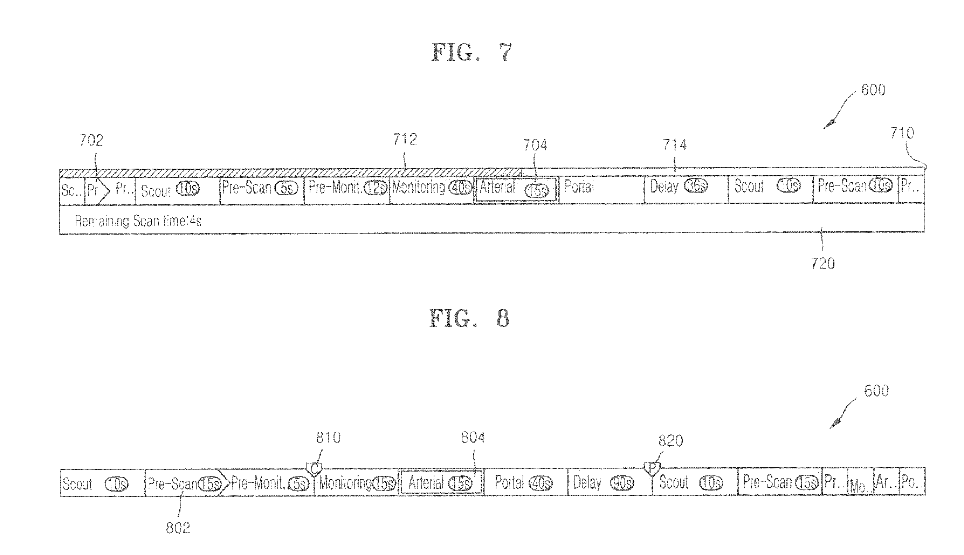

For example, the time information may include information about the time when a predetermined execution procedure begins or the time when the predetermined execution procedure finishes, and a total time for finishing the execution procedures. The time information may include information about the remaining time until a predetermined execution time is finished. The state display bar displaying the time information will be described in more detail below with reference to FIGS. 4 and 7.

The state display bar according to the present exemplary embodiment may include icons for guiding user operations for acquiring the medical images. The icons may be displayed between the buttons corresponding to the execution procedures that are performed before and after the current user operation. The state display bar including the icons for guiding the user operations will be described in more detail below with reference to FIG. 8.

In operation S230, the medical image display apparatus displays the state display bar generated in the operation S220.

When one of the plurality of buttons included in the state display bar is selected, the medical image display apparatus 120 may further display on a screen medical image acquisition information corresponding to the selected button.

The medical image acquisition information corresponding to the selected button may include at least one of the medical image acquired in the execution procedure corresponding to the selected button, state information relating to the execution procedure corresponding to the selected button, and a user interface relating to the execution procedure corresponding to the selected button.

The medical image display apparatus 120 according to the present exemplary embodiment may arrange the plurality of buttons corresponding to the plurality of execution procedures for acquiring the medical image according to an order of executing the processes.

Therefore, the medical image display apparatus 120 may provide the user with information relating to the medical image acquisition according to a timeline of the processes of acquiring the medical image.

The medical image display apparatus 120 according to the present exemplary embodiment may distinguish the button corresponding to the reference execution procedure from the other buttons. The medical image display apparatus 120 may display the medical image acquisition information so that the user may intuitively distinguish the reference execution procedure from all execution procedures for acquiring the medical image.

If the buttons for providing the information about the execution procedures for acquiring the medical image are not simultaneously displayed on one screen, the user needs to search for information corresponding to a certain execution procedure, and an operation for finding the button corresponding to the execution procedure from among the plurality of arranged buttons, for example, a mouth scrolling operation, is needed.

However, the medical image display apparatus 120 according to the present exemplary embodiment may vary the length of the button corresponding to each of the execution procedures according to the user's interest. Therefore, the medical image display apparatus 120 may change the lengths of the buttons, and thus, the plurality of buttons corresponding to the plurality of execution procedures may be displayed simultaneously on the screen irrespective of the number of buttons.

Therefore, the medical image display apparatus 120 according to the present exemplary embodiment may display the plurality of execution procedures for acquiring the medical image, that is, from the starting process to the ending process, at one screen, and thus, an additional user operation for finding the information about the execution procedure that is not displayed on the screen, for example, the mouth scrolling operation, may be avoided.

The medical image display apparatus 120 may determine the execution procedure that is currently performed by the medical image acquisition apparatus 110 or the execution procedure selected by the user as the execution procedure that the user is interested in. The medical image display apparatus 120 may enlarge the button corresponding to the execution procedure that the user is interested in to be bigger than the other buttons corresponding to the other execution procedures. Therefore, more information about the execution procedure that the user is interested in may be displayed. The execution procedure that the user is interested in may correspond to the reference execution procedure that is described above.

For example, according to the medical image display apparatus 120 of the present exemplary embodiment, the button corresponding to the execution procedure that the user is interested in may have a length that is longer than the other buttons corresponding to the execution procedures that the user is not interested in. Therefore, the entire name of the execution procedure that the user is interested in may be displayed on the corresponding button. In addition, names of the other execution procedures may be partially displayed on the buttons.

The medical image display apparatus may display where the execution procedure that the user is interested in is located among the plurality of execution procedures on the display bar, and at the same time, may display medical image acquisition information relating to the execution procedure that the user is interested in on a predetermined region of the screen. Therefore, the user may intuitively recognize the execution procedure corresponding to the medical image acquisition information displayed on the screen.

FIG. 3 is a flowchart illustrating a method of displaying medical image acquisition information according to a selection of the user, according to the exemplary embodiment.

In operation S342, the medical image display apparatus 120 may identify the button selected by the user.

The medical image display apparatus 120 may recognize a user input for selecting at least one of the buttons included in the state display bar displayed on the screen.

The medical image display apparatus 120 may include a user input unit (not shown) for recognizing the selection of the user. The user input unit may be a unit for inputting data for controlling the medical image display apparatus from the user. For example, the user input unit may include a keypad 122, a mouth 124, a dome switch, a touch pad 126 (a capacitive overlay type, a resistive overlay type, an infrared beam type, a surface acoustic wave type, an integral strain gauge type, and a piezoelectric type), a jog wheel, or a jog switch; however, an exemplary embodiment is not limited thereto.

In operation S344, the medical image display apparatus 120 may change lengths of the button that is selected and the buttons adjacent to the selected button.

The buttons adjacent to the selected button may include the buttons corresponding to the predetermined number of execution procedures that are performed before and/or after the execution procedure corresponding to the selected button. The predetermined number of buttons may be stored in the DB 560 in advance or may be stored according to the user input.

For example, the medical image display apparatus 120 may increase the lengths of the buttons adjacent to the selected button to be longer than the other buttons so that the entire titles of the execution procedures may be displayed on the buttons. Titles of the other execution procedures may be partially displayed, for example, only two characters from the entire title may be displayed.

In operation S346, the medical image display apparatus 120 may display medical image acquisition information corresponding to the button selected in the operation S342.

For example, the medical image display apparatus 120 may display at least one of the medical image acquired in the execution procedure corresponding to the selected button, state information relating to the execution procedure corresponding to the selected button, and a user interface relating to the execution procedure corresponding to the selected button.

FIG. 4 is a flowchart illustrating a method of displaying medical image acquisition information by the medical image display apparatus 120 based on the execution procedure performed in the medical image system 100, according to the exemplary embodiment.

In operation S442, the medical image display apparatus 120 may identify the execution procedure that is currently performed in the medical image system 100.

For example, the medical image display apparatus 120 may identify the execution procedure that is currently performed in the medical image acquisition apparatus 110 via communication with the medical image acquisition apparatus 110 that is connected to the medical image display apparatus 120.

In operation S444, the medical image display apparatus 120 may change the lengths of the button corresponding to the execution procedure identified in operation S442 and the adjacent buttons.

The buttons adjacent to the button corresponding to the identified execution procedure may include the buttons corresponding to the predetermined number of execution procedures of the sequence performed before and/or after the execution procedure that is currently performed. The predetermined number of buttons may be stored in the DB 560 in advance or may be stored according to the user input.

For example, the medical image display apparatus 120 may increase the lengths of the button corresponding to the execution procedure that is currently performed and of the adjacent buttons to be longer than those of the other buttons so that the entire titles of the execution procedures may be displayed on the buttons. Titles of the other execution procedures may be partially displayed, for example, only two characters in the entire title may be displayed.

In operation S446, the medical image display apparatus 120 may display time information relating to the execution procedure that is currently performed.

The time information relating to the execution procedure may be obtained from the DB 560. The medical image display apparatus 120 may acquire the time information relating to the execution procedure through communication with the medical image acquisition apparatus 110. The medical image display apparatus 120 may generate time information by combining the information acquired from the DB 560 or the information acquired from the medical image acquisition apparatus 110.

For example, the medical image display apparatus 120 may acquire a time when the execution procedure that is currently performed begins from the medical image acquisition apparatus 110, and may calculate the time passed after the beginning of the currently performed execution procedure. The medical image display apparatus 120 may calculate a remaining time until the corresponding execution procedure is finished, based on the time that has passed from the beginning of the currently performed execution procedure.

In order to display the time information, the state display bar may include a time display bar for displaying the time information as a bar-type image, or a time display area for displaying the time information by letters. For example, the time display bar or the time display area may be located on an upper or lower portion of the plurality of buttons that are arranged in a row in the state display bar; however, an exemplary embodiment is not limited thereto.

As an example, the time display bar may display at least one of a total time for processing the plurality of executing processes, elapsed time from the beginning of the plurality of execution procedures to the current execution procedure, and remaining time until the plurality of execution procedures are finished.

As another example, the time display bar may display at least one of a total time for processing a predetermined execution procedure from among the plurality of execution procedures, an elapsed time from the beginning of the corresponding execution procedure to the current point, and a remaining time until the corresponding execution time is finished.

A total time of the execution procedure corresponding to at least one button from among the plurality of buttons included in the state display bar may be displayed.

For example, the time display bar may display a total time of the execution procedure that is currently performed by the medical image acquisition apparatus 110. The time display area may display a remaining time until the execution procedure that is currently performed by the medical image acquisition apparatus 110 is finished.

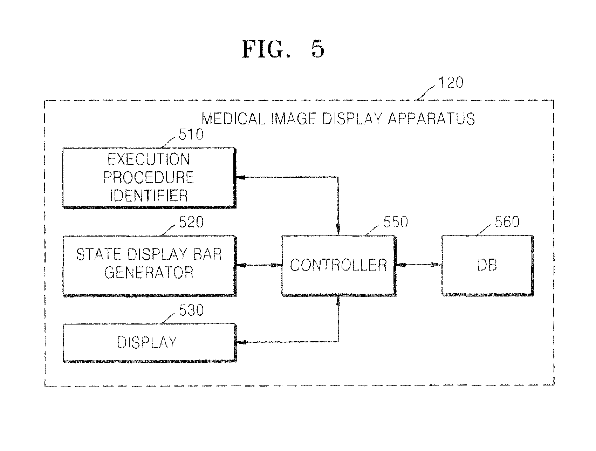

FIG. 5 is a block diagram of the medical image display apparatus 120 according to the exemplary embodiment.

The medical image display apparatus 120 according to the present exemplary embodiment may provide the user with the medical images obtained from an object. The medical image display apparatus 120 may control the medical image acquisition apparatus 110 in a wired or wireless manner.

The medical image display apparatus 120 of the present exemplary embodiment may be realized variously. For example, the medical image display apparatus 120 may be a mobile terminal as well as a fixed terminal. Examples of the mobile terminal may include a laptop computer, a personal digital assistant (PDA), or a tablet PC. The medical image display apparatus 120 may be included in a medical image acquisition apparatus (not shown).

Each of the components in the medical image display apparatus 120 according to the present exemplary embodiment may be configured to perform each of the processes for displaying medical image acquisition information described with reference to FIG. 2. Therefore, the description about the medical image acquisition information display apparatus with reference to FIG. 2 may be applied to the medical image display apparatus 120 of FIG. 5.

The medical image display apparatus 120 of the present exemplary embodiment may include an execution procedure identifier 510, a state display bar generator 520, a display 530, and a DB 560.

The execution procedure identifier 510 identifies the plurality of execution procedures that are necessary for acquiring medical images about the object.

The state display bar generator 520 generates a state display bar relating to the plurality of execution procedures identified by the execution procedure identifier 510.

The state display bar generator 520 generates the state display bar including a plurality of buttons for displaying the medical image acquisition information relating to the plurality of execution procedures. The state display bar generator 520 determines a reference execution procedure from among the plurality of execution procedures, and generates the state display bar so that a button corresponding to the determined reference execution procedure may have a length that is different from those of the other buttons.

The display 530 displays information relating to the medical image acquisition.

For example, the display 530 displays the state display bar generated by the state display bar generator 520 on a screen. The display 530 may display information processed by the medical image display apparatus 120. For example, the display 530 may display the state information relating to the execution procedures for acquiring the medical images, a UI or a GUI relating to setting of functions, and a medical image of the object.

If a display panel and a touch pad that will be described below configure a layered structure to form a touch screen, the display 530 may be used as an input device, as well as the output device.

The display 530 may include at least one of a liquid crystal display (LCD), a thin film transistor-liquid crystal display, an organic light emitting diode, a flexible display, a three-dimensional (3D) display, and an electrophoretic display.

A controller 550 controls operations of the medical image display apparatus 120, and also controls the execution procedure identifier 510, the state display bar generator 520, the display 530, and the DB 560 for displaying the information relating to the medical image acquisition.

The DB 560 stores various pieces of information for the medical image display apparatus 120 to display the information relating to the medical image acquisition. For example, the DB 560 may store titles of the plurality of execution procedures for acquiring medical images of the object, time information, status information, function setting information, and acquired medical images according to the object; however, an exemplary embodiment is not limited thereto.

As shown in FIG. 5, the DB 560 may be accommodated in the medical image display apparatus 120. Otherwise, the DB 560 may be located at outside of the medical image display apparatus 120 and provide the medical image display apparatus 120 with the stored information via wired or wireless communication.

Hereinafter, the method of displaying the information relating to the medical image acquisition by the medical image display apparatus 120 will be described in detail below with reference to FIGS. 6 through 12.

As shown in FIGS. 6 through 12, the medical image display apparatus 120 may display a state display bar 600 including a plurality of buttons corresponding to the plurality of execution procedures that are arranged in a sequence, for acquiring medical images. The state display bar 600 displayed by the medical image display apparatus 120 may include the plurality of buttons that are arranged in a row. Titles of the execution procedures respectively corresponding to the buttons may be displayed on the corresponding buttons.

Referring to FIGS. 6 through 12, the plurality of execution procedures for acquiring the medical image may include a scout scanning process, a pre-scanning process, a pre-monitoring process, a monitoring process, an arterial imaging process, a portal imaging process, and a delay process; however, an exemplary embodiment is not limited thereto.

The state display bar 600 shown in FIGS. 6 through 12 is displayed horizontally on the screen; however, an exemplary embodiment is not limited thereto. For example, the medical image display apparatus 120 may display the state display bar 600 including the plurality of buttons arranged vertically on the screen.

FIGS. 6A and 6B are diagrams showing an example of the state display bar for displaying information relating to the medical image acquisition, according to the exemplary embodiment,

A button 612 corresponds to the execution procedure that is currently performed in the medical image system 100. The medical image display apparatus 120 identifies the execution procedure that is currently performed, and may increase a length of the button 612 corresponding to the currently performed execution procedure to be longer than those of the other buttons 616.

The medical image display apparatus 120 may change a shape of the button 612 to be different from those of other buttons 614 and 616 that correspond to the other execution procedures that are not performed currently.

The medical image display apparatus 120 may increase lengths of the buttons 614 corresponding to four execution procedures before or after the execution procedure that is currently performed to be longer than those of the other buttons 616.

The button 612 corresponding to the execution procedure that is currently performed may have a length that is long enough to display the entire title of the corresponding execution procedure on the button 612. The lengths of the buttons 614 adjacent to the button 612 may be long enough to display the entire or nearly entire titles of the corresponding execution procedures on the buttons 614. Only the first two characters in the titles of the corresponding execution procedures may be displayed on the buttons 616.

The medical image display apparatus 120 may display the execution procedure that is currently performed from among the entire execution procedures so that the user may check the currently performed execution procedure intuitively. The medical image display apparatus 120 may provide much information regarding the execution procedure currently performed and the adjacent execution procedures while displaying the plurality of buttons corresponding to the plurality of execution procedures on a screen. That is, the titles of the execution procedure that is currently performed and the adjacent execution procedures of the sequence may be displayed in their entireties on the state display bar 600.

In FIG. 6B, a button 622 corresponds to the execution procedure that is currently performed in the medical image system 100. The medical image display apparatus 120 may change a shape of the button 622 corresponding to the execution procedure that is currently performed to be different from those of buttons 624, 626, and 628 corresponding to the execution procedures that are not currently performed.

The button 628 is selected by the user. For example, the user clicks or scrolls a mouse over the button 628 to select the button 628. The medical image display apparatus 120 identifies the button 628 selected by the user, and may increase a length of the button 628 to be longer than those of the other buttons 622 and 626 that are not selected.

The medical image display apparatus 120 may increase lengths of buttons 624 corresponding to four execution procedures of the sequence before and after the execution procedure corresponding to the selected button 628 to be longer than those of the other buttons 622 and 626.

The lengths of the selected button 628 and the adjacent buttons 624 may be long enough to display entire titles of the corresponding execution procedures on the buttons 628 and 624. For example, only the first two characters in the entire titles may be displayed on the other buttons 622 and 626.

Therefore, the medical image display apparatus 120 may display the buttons so that the user may intuitively recognize the execution procedure that is selected from among the plurality of execution procedures. The medical image display apparatus 120 may provide a lot of information regarding the execution procedure currently performed and the adjacent execution procedures, while displaying the plurality of buttons corresponding to the plurality of execution procedures on a screen. That is, the titles of the execution procedure that is currently performed and the adjacent execution procedures may be displayed entirely on the state display bar 600.

The medical image display apparatus 120 may change the lengths of the buttons based on one of the execution procedure that is selected by the user and the execution procedure that is currently performed. For example, as shown in FIG. 6B, the medical image display apparatus 120 may change the lengths of the buttons based on the execution procedure selected by the user.

For example, if the user may select a button that is not the button corresponding to the execution procedure that is currently performed, the medical image display apparatus 120 may change the lengths of the buttons on the state display bar 600 based on the selected button. That is, the medical image display apparatus 120 may change the lengths of the selected button and the buttons adjacent to the selected buttons to be different from those of the other buttons.

As another example, if the button selected by the user is different from the button corresponding to the execution procedure that is currently performed, the medical image display apparatus 120 may change the lengths of the buttons on the state display bar based on the execution procedure that is currently performed. That is, the lengths of the button corresponding to the execution procedure that is currently performed and the lengths of the adjacent buttons may be changed to be different from those of the other buttons.

After changing the lengths of the buttons on the state display bar 600 based on the button selected by the user, the medical image display apparatus 120 may change the lengths of the buttons on the state display bar based on the execution procedure that is currently performed if there is no input from the user for a predetermined time period. That is, when a predetermined time period has passed after the execution procedure selected by the user is determined as the reference execution procedure, the medical image display apparatus 120 may change the reference execution procedure into the execution procedure that is currently performed by the medical image acquisition apparatus 110.

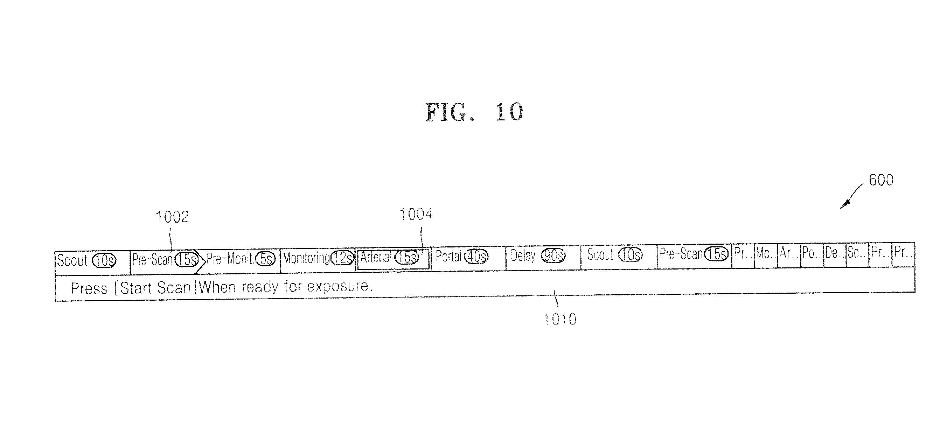

If information about the execution procedure that is currently performed is updated, the medical image display apparatus 120 may determine the execution procedure that is currently performed as the reference execution procedure. If there is an update of the information about the execution procedure that is currently performed after changing the lengths of the buttons in the state display bar based on the execution procedure selected by the user, the execution procedure that is currently performed may be determined as the reference execution procedure. For example, if a next execution procedure is performed or if an alarm message about the execution procedure that is currently performed is displayed, the lengths of the buttons in the state display bar may be changed based on the execution procedure that is currently performed. The alarm message displayed on the state display bar will be described in detail with reference to FIG. 10.

FIG. 7 shows an example of the state display bar displaying time information according to the exemplary embodiment.

In FIG. 7, a button 702 corresponds to the execution procedure that is currently performed in the medical image system 100. A button 704 is selected by the user. Referring to FIG. 7, the state display bar 600 generated and displayed by the medical image display apparatus 120 may display time information relating to at least one of the plurality of execution procedures.

The state display bar 600 shown in FIG. 7 includes a time display bar 710 for displaying the time information as a bar type image, and a time display area 720 for displaying the time information as letters.

The time display bar 710 may display a total time of the execution procedure that is currently performed by the medical image acquisition apparatus 110. The time display bar 710 may include a region 712 for displaying elapsed time from the beginning of an execution of the execution procedures of a sequence to the present, and a region 714 for displaying remaining time from the present to the finishing of the execution procedures. The regions 712 and 714 may be distinguished from each other by differentiating contrasts, colors, or patterns.

The time display area 720 displays remaining time until the execution procedure that is currently performed is finished. Referring to FIG. 7, the time display area 720 displays 'Remaining Scan time: 4s'. The user may know that the remaining time until the execution procedure that is currently performed is finished is 4 seconds from the information displayed in the time display area 720.

As shown in FIG. 7, the medical image display apparatus 120 may display the time information relating to each execution procedure on the corresponding button. For example, the total time of the execution procedure corresponding to the button 704 may be 15 seconds.

FIG. 8 shows an example of a state display bar including icons for guiding operations of the user, according to the exemplary embodiment.

In FIG. 8, a button 802 corresponds to the execution procedure that is currently performed in the medical image system 100. A button 804 is selected by the user.

Referring to FIG. 8, the state display bar 600 generated and displayed by the medical image display apparatus 120 may include icons 810 and 820 for guiding operations of the user, which are needed for acquiring the medical image. For example, the user operations for acquiring the medical image may include injection of a contrast medium or changing a patient's position.

The icon 810 guides the user to inject the contrast medium into the object. The icon 820 guides the user to vary the patient's position. The icons 810 and 820 for guiding the user operations may be displayed between the buttons corresponding to the execution procedures before and after the user operation.

The medical image display apparatus 120 displays the icons 810 and 820 as shown in FIG. 8 so that the user may intuitively recognize when the user operations have to be performed between the execution procedures.

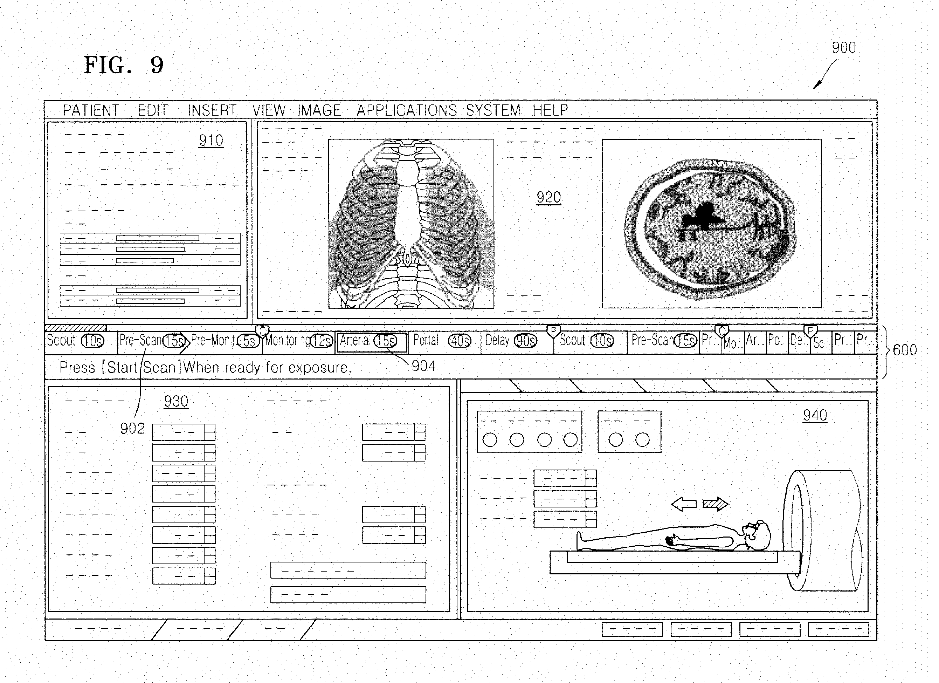

FIG. 9 shows an example a screen 900 displaying medical image acquisition information according to the exemplary embodiment.

In FIG. 9, a button 902 corresponds to the execution procedure that is currently performed in the medical image system 100. A button 904 is selected by the user.

When one of the plurality of buttons included in the state display bar 600 is displayed on the screen, the medical image display apparatus 120 may display medical image acquisition information corresponding to the selected button 904.

The medical image acquisition information displayed on the screen may include an image acquired in the execution procedure corresponding to the selected button 904, state information of the medical image system 100 relating to the execution procedure corresponding to the selected button 904, state information of the object relating to the execution procedure corresponding to the selected button 904, and user interface relating to the execution procedure corresponding to the selected button 904.

Referring to FIG. 9, for example, information about the object may be displayed in a region 910. The information about the object may include biographical data, the medical history of the object, and information about a part to be imaged.

A medical image may be displayed on the region 920. The medical image displayed on the region 920 may include an image acquired in the execution procedure corresponding to the selected button 904, or reference image for diagnosing disease.

Information about a status of the medical image system 100 may be displayed in a region 930. The information about the status of the medical image system 100 may include set values about the medical image acquisition apparatus 110, set values about the medical image display apparatus 120, and various measured values for determining whether the medical image system 100 normally operates.

State information of the object may be displayed in a region 940. For example, the state information of the object may include information representing movement of the table for supporting the object, information about laying orientation of the object, and relative position information with respect to the medical image acquisition apparatus 110.

FIG. 10 shows an example of a state display bar 600 including a notification message relating to execution procedures, according to the exemplary embodiment.

In FIG. 10, a button 1002 corresponds to the execution procedure that is currently performed in the medical image system 100. A button 1004 is selected by the user.

As shown in FIG. 10. the state display bar 600 may include a region 1010 for displaying a notification message relating to a predetermined execution procedure. The notification message displayed in the region 1010 may relate to the execution procedure that is currently performed.

For example, the notification message may guide an operation that has to be performed by the user or prepared by the user after the currently performed execution procedure or before the next execution procedure. The notification message may include information about warnings.

Referring to FIG. 10, the region 1010 displays the message 'Press [Start Scan] when ready for exposure'. Therefore, the notification message displayed in the region 1010 may notify the user to push the [start scan] button to proceed to the next process when the object is ready for exposure.

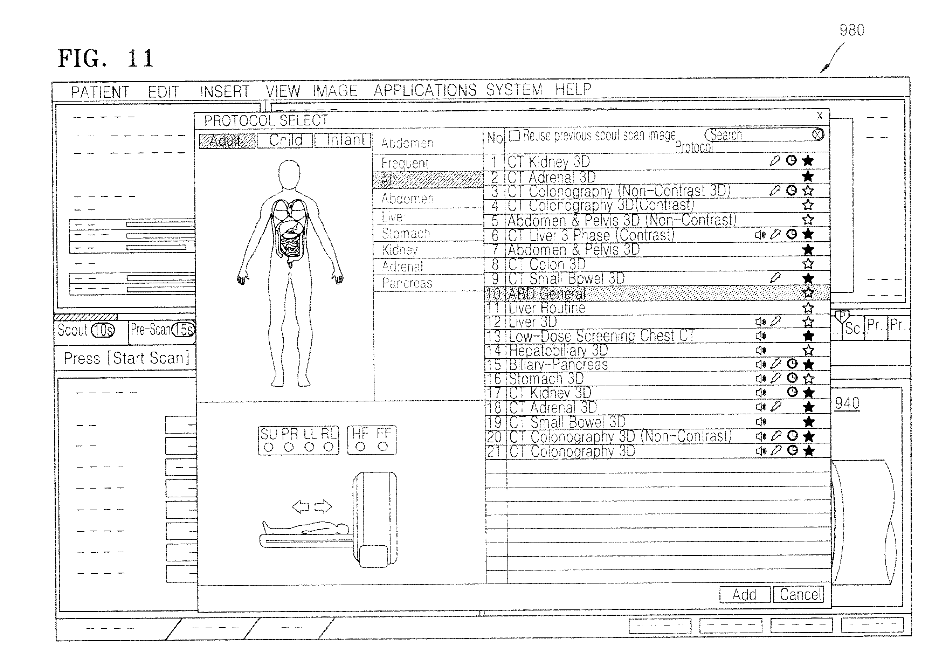

FIG. 11 is a diagram showing an example of a GUI 980 for identifying a plurality of execution procedures for acquiring a medical image according to the exemplary embodiment.

The medical image display apparatus 120 may acquire information about the object. The medical image display apparatus 120 may acquire information about the object from the user's input. The medical image display apparatus 120 may search for the medical image acquisition method stored in the DB 560 based on the acquired information about the object. If a plurality of medical image acquisition methods is found, the medical image display apparatus 120 may display a user interface so that the user may select one of the plurality of medical image acquisition methods.

For example, as shown in FIG. 11, the user may select a predetermined part of the object via the user interface displayed on the screen.

The medical image display apparatus 120 may search for the medical image acquisition method for the selected part of the object from the DB 560. If a plurality of medical image acquisition methods is found, the user may select one of the plurality of medical image acquisition methods via the user interface displayed on the screen.

The medical image display apparatus 120 may search for a plurality of execution procedures for acquiring the medical image of the selected part of the object from the DB 560. The user may select execution procedures that may be considered unnecessary from among the plurality of execution procedures via the user interface displayed on the screen. The medical image display apparatus 120 may control the medical image acquisition apparatus 110 so as not to perform the execution procedures that are determined to be unnecessary by the user.

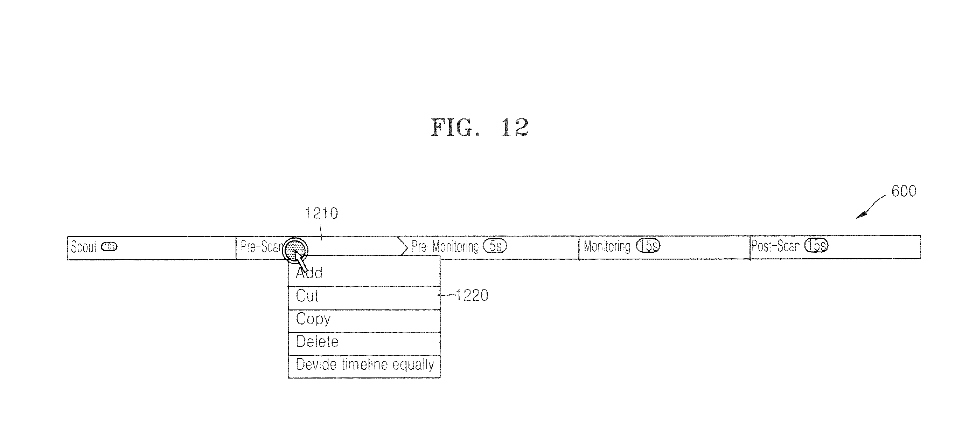

FIG. 12 shows an example of a user interface for changing the state display bar 600 according to the exemplary embodiment. Each of the plurality of execution procedures included in the state display bar may be changed.

As shown in FIG. 12, the medical image display apparatus 120 provides a user interface 1220 for changing the state display bar 600. For example, when the user locates a cursor on a button 1210 and clicks the button 1210, the user interface 1220 may be displayed.

The user interface 1220 about the button 1210 may include menus for cutting the button 1210 off, copying the button 1210, or deleting the button 1210. The user interface 1220 may include a menu for adding a new button. The user interface 1220 may also include a menu for changing the lengths of the plurality of buttons to be the same as each other.

The medical image display apparatus 120 may delete a button on the state display bar in order to delete the execution procedure corresponding to the deleted button from the entire processes for acquiring the medical image. The medical image display apparatus 120 may add a new button to the state display bar in order to add an execution procedure corresponding to the button to the entire processes for acquiring the medical image.

Exemplary embodiments can also be embodied as computer-readable codes on a non-transitory computer-readable recording medium. The non-transitory computer-readable recording medium is any data storage device that can store data which can be thereafter read by a computer system, and may include volatile and non-volatile media, and separatable and non-separatable media. The non-transitory computer-readable recording medium may include computer storage media and communication media. The computer storage medium may include volatile and non-volatile, separatable and non-separatable media realized by an arbitrary method or technology, such as computer-readable commands, data structure, program modules, and other types of data. The communication medium may typically include data of modulated data signals or other transmission mechanisms such as computer-readable commands, data structures, program modules, or carrier waves, and may include an arbitrary information transmission medium.

According to the exemplary embodiments, the information relating to the medical image acquisition required by the user may be provided intuitively.

According to the exemplary embodiments, the information relating to the medical image acquisition is provided based on the plurality of execution procedures for acquiring the medical image, and thus, the user may precisely recognize the information about each of the execution procedures.

The described-above exemplary embodiments and advantages are merely exemplary and are not to be construed as limiting. The present teaching can be readily applied to other types of apparatuses. The description of exemplary embodiments is intended to be illustrative, and not to limit the scope of the claims, and many alternatives, modifications, and variations will be apparent to those skilled in the art.

## Claims

1. A method of displaying information relating to medical image acquisition, the method comprising:
identifying execution procedures for acquiring medical images of an object;
generating a state display bar comprising buttons relating to the execution procedures: and
displaying the state display bar on a screen,
wherein the buttons are arranged on the state display bar in correspondence to the execution procedures of a sequence, and
the button corresponding to one of the execution procedures has a length that is different from lengths of all or some of other buttons corresponding to other execution procedures.

2. The method of claim 1, wherein the one of the execution procedures is an execution procedure selected by a user.

3. The method of claim 2, wherein the other execution procedures include adjacent execution procedures which are adjacent in the sequence to the execution procedure selected by the user, and
the lengths of the buttons of the adjacent execution procedures are different from the lengths of the buttons corresponding to the execution procedures which are distal, in the sequence, to the execution procedure selected by the user.

4. The method of claim 1, wherein the one of the execution procedures is an execution procedure that is currently performed.

5. The method of claim 4, wherein the other execution procedures include adjacent execution procedures which are adjacent, in the sequence, to the execution procedure that is currently performed, and
the lengths of the buttons of the adjacent execution procedures are different from the lengths of the buttons corresponding to the execution procedures which are distal, in the sequence, to the execution procedure that is currently performed.

6. The method of claim 4, wherein the state display bar further comprises a time display bar configured to display a total time of the execution procedure that is currently performed.

7. The method of claim 4, wherein the state display bar further comprises a region configured to display a remaining time until the execution procedure that is currently performed is finished.

8. The method of claim 1, wherein the state display bar further comprises an entire time display bar configured to display at least one of:
a total time for performing the execution procedures,
an elapsed time from a beginning of an execution of the execution procedures to a point of time at which the first execution procedure is currently performed, and
a remaining time until the execution of the execution procedures is finished.

9. The method of claim 1, wherein the state display bar comprises icons configured to guide operations of a user for acquiring a medical image.

10. The method of claim 1, wherein the button corresponding to the one of the execution procedures has at least one of a different color and a different shape from the other buttons corresponding to the other execution procedures.

11. The method of claim 1, wherein the buttons arranged on the state display bar are displayed on one screen.

12. The method of claim 1, further comprising:
receiving a selection of one of the buttons; and
displaying medical image acquisition information corresponding to the selected button on the screen.

13. The method of claim 12, wherein the medical image acquisition information displayed on the screen comprises at least one of:
an image acquired in the execution procedure corresponding to the selected button,
state information relating to the execution procedure corresponding to the selected button, and
a user interface relating to the execution procedure corresponding to the selected button.

14. A medical image display apparatus comprising:
an execution procedure identifier configured to identify execution procedures for acquiring a medical image of an object;
a state display bar generator configured to generate a state display bar comprising buttons relating to the execution procedures; and
a display configured to display the generated state display bar on a screen,
wherein the buttons are arranged on the state display bar in correspondence to the execution procedures of a sequence, and
one of the buttons corresponding to one of the execution procedures has a length that is different from lengths of all or some of other buttons corresponding to other execution procedures.

15. A computer-readable recording medium having embodied thereon a computer program for executing the method of claim 1 on a computer.
